# EUROPEAN PATENT APPLICATION

(11) **EP 2 468 111 A1**
(43) Date of publication of application: **27.06.2012**
(21) Application number: 11183448.7
(22) Date of filing: 30.09.2011
(51) Int. Cl.: A23L 1/30, A23K 1/16, A23L 1/00, A61P 27/12, A23L 1/302, A23L 1/275, A61K 31/015

(54) **Compositions and applications of carotenoids of improved absorption and bioavailability**

(30) Priority: 21.12.2010 US 974169
(71) Applicant: Torres-Quiroga, Jose-Odon, 66290 Garza Garcia, Neuvon Leon (MX)
(72) Inventor: Torres-Quiroga, Jose-Odon, 66290 Garza Garcia , Nuevon Leon (MX); Montoyta-Olvera, Ricardo, 64150 Monterrey N.L. (MX)
(74) Representative: Colens, Alain M.G.M.

(57) **Abstract**

Micro micellar form of carotenoids or xanthophylls, which show improved absorption and biovailability by living organisms, providing higher levels of carotenoids in the blood stream, and consequently are deposited at the target tissues at a faster rate, and more efficiently than crystalline carotenoids, which does not contain any crystal forms of carotenoids, and which are obtained as micro micelles after melting Lutein diacetate or Lutein dipropionate in their natural original lipid vegetable matrix, in the presence of lipids, phospholipids, fatty acids, emulsifiers and moisture, and are ingested in a dosage between 2 to 50 mg, to provide an absorption of the carotenoid solubilizate which is at least 20% above than that of crystalline carotenoids, and provides a macular pigment deposit which exceeds at least 10% of Macular Pigment Optical Density, than the deposition obtained by ingesting crystalline Lutein. Thus preventing tissue degeneration, UV light damage to skin and the retina, and in general to act as more effective and efficient antioxidants than when administered in crystalline form.

## Description

### BACKGROUND OF THE INVENTION

### A. - FIELD OF THE INVENTION

The present invention is related to micro micellar form of carotenoids or xanthophylls, which show to be readily absorbed by living organisms, and therefore is more bioavailable than crystalline forms of carotenoids. Such improved absorption and biovailability provides higher levels of carotenoids in the blood stream, and consequently the carotenoids are deposited at the target tissues at a faster rate, and more efficiently than crystalline carotenoids.

### B. - DESCRIPTION OF THE RELATED ART

It has been the objective of many researchers, to improve the absorption and bioavailability of carotenoids. Xanthophylls contained in many green leafy vegetables as well as in fruits are quite stable, but do have a limited bioavailability. On the other hand when vegetables are cooked and the chromoplast tissues are broken because of temperature, the carotenoids are more bioavailable, but their stability is decreased. Therefore the best balance between stability and availability is very desirable. An outstanding example of stability and bioavailability of Lutein and Zeaxanthin is the particular form in which they are found in the egg yolk. Lutein and Zeaxanthin occur in the egg yolk as microscopic micro micelles, not in crystalline form.

Many attempts have been made to improve the bioavailability and absorption of carotenoids. Among the most recent are:
US Patent 2,861,891 issued to Bauernfeind and Howard describes a process to obtain a dry powder, obtaining a supersaturated carotene solution by heating a vegetable oil that afterwards is dispersed in an aqueous gelable colloid solution, and converting the emulsion thus formed into a dry particulate form. Such process involves heating the carotenoids in vegetable oil in order to improve the solubility of the carotenes, lycopene, Lutein, Zeaxanthin, Cryptoxanthin, bixin and methyl bixin, and avoiding the precipitation of carotenoids crystals by incorporating a gelable colloid. A further emulsification step follows in an aqueous colloidal solution that forms a gel capable of producing a dry powder after a spray drying process. The product obtained is a microcrystalline dispersion in edible oil used to impart color to margarine, fruits and vegetables in which the βcarotene occurs in the form of micro dispersion in a protective hydrophilic colloid.
US Patent 3,523,138 issued to Grant describes the improved bioavailability of carotenoids in poultry, after a saponification reaction has taken place in order to free the xanthophylls from the long chain fatty acid esters.
US Patent 3,535,426 issued to Hawks discloses that a saponified mixture of xanthophylls become more stable, and consequently more bioavailable, when admixed with a fat and ethoxyquinolein.

Cathrein describes in US Patent 5,364,563 a process for producing powdered carotenoid preparations by obtaining a suspension in oil that is brought in contact with superheated steam, producing an emulsion that is spray dried.

Eugster et al, in US Patents 5,496,813 and 5,536,504 obtain ultra micro emulsions that form spontaneously dispersible concentrates containing xanthophylls esters that have anti tumor activity.

Gellenbeck in US Patent 5,827,539 obtains a finely ground mixture of carotenoids with an oil and a spray dried encapsulated form that is water dispersible.

Luddecke, et al, in US Patent 5,863,953 describes the obtention of an oil dispersion of carotenoids, which is used to prepare a double dispersion system with particles sizes of 100 microns, stabilized by a protective colloid and emulsifiers.

Sanders and Herink published in WO9947001 the increased bioavailability of Lutein and Zeaxanthin in humans and poultry using isolecithin and lecithin.

Kolter et al, describe in US Patent 5,891,907 stable aqueous solubilizates of carotenoids and vitamins, in which the carotenoids and the water insoluble vitamins with the aid of a nonionic emulsifier, yield a micelle, which particles are smaller than 100 nm.

Luddecke et al, describe in US Patent 5,895,659 the preparation of finely dispersed carotenoids or retinoid suspensions by dissolving the carotenoid or retinoid in a volatile, water-miscible organic solvent, under elevated pressures, and immediately after 10 seconds, mix the solution with an aqueous medium containing an emulsifier.

Schweikert et al in US Patent 5,925,684 describe a stable oil in water emulsion consisting of an aqueous phase and an oil phase which is very finely dispersed by means of an emulsifier, and wherein the carotenoid is present in the oil phase in a concentration above the saturation solubility of the carotenoid in the oil at room temperature.

Auweter et al, describe in US Patent 5,968,251 the preparation of coldwater dispersible powders by preparing a molecular-disperse solution of a carotenoid, with or without an emulsifier and/or edible oil, in a volatile, water-miscible organic solvent at elevated temperature and adding an aqueous solution of a protective colloid; whereupon the hydrophilic solvent is transferred into the aqueous phase, and the hydrophobic phase of the carotenoid results as a nanodisperse phase, removing the solvent by heating the hydrosol and converting it in a water dispersible dry powder.

Gellenbeck in US Patent 5,976,575 describes the grinding of a mixture of carotenoids and oil to reduce the carotenoids particle size, emulsifying the mixture with an encapsulating mixture and drying the emulsion.

Handelman in US Patent 6,075,058 describes the composition of Lutein and Zeaxanthin along with cholesterol, olive oil, egg yolk phospholipids, alpha tocopherol and aqueous sodium chloride. The mixture is prepared by mixing the lipid ingredients into ethanol, evaporating the ethanol, and dispersing the lipids as an emulsion in the sodium chloride solution.

Kowalski et al, describe in US Patent 6,093,348 a method for the manufacture of carotenoid powders, weherein an aqueous suspension of the carotenoid is heated to melt the carotenoid in the presence of a surfactant and a protective colloid under high temperatures and high pressures (HTHP process). The suspension is then homogenized under high pressure to form an emulsion. And the resulting emulsion is dried to obtain the carotenoid powder.

Schliapalius in US Patent 6,132,790 describes a composition of a carotenoid in oil, a dispersion of a water dispersible matrix and a stabilizer, and a non-oil solvent and an emulsifier, all of natural sources.

Koguchi, et al describe in US Patent 6,261,622 a method to provide a water-dispersible carotenoid preparation which can be added to various aqueous compositions with retaining dispersion stability even at low temperature, by dispersing pulverized carotenoids crystals with soybean extract fibers as emulsion stabilizer.

Bewert et al, in US Patent 6,328,995 describe a procedure to stabilize dry powders which are insoluble in hot water and which contain one or more lipid soluble vitamins or carotenoids, formed in an aqueous dispersion containing a protein, a sugar and potassium and/or sodium phosphates.

Stein et al, describe in US Patent 6,406,735 a process for the preparation of a pulverous composition of a finely divided carotenoid or retinoid comprising; forming a suspension of the active ingredient in a water-immiscible organic solvent containing an antioxidant and/or an oil, feeding the suspension through a heat exchanger and heating the suspension to a high temperature for a 5 seconds residence time, rapidly mixing the solution with an aqueous swellable colloid and further removing the organic solvent to obtain the pulverous preparation, all steps processed in a continuous sequence.

Guerra-Santos, et al describe in US Patent 6,936,279 the obtention of microcrystalline form of carotenoids, particularly zeaxanthin, in an oily carrier. The "coarse-grained" carotenoids is dissolved in a suitable solvent as tetrahydrofuran, and mixed with a vegetable oil and an emulsifier. The mixture is injected along with an inert gas into a vacuum chamber in order to remove the solvent in a flash manner, not allowing the carotenoids crystals to grow. They obtain a microcrystalline suspension in the oil carrier.

Eidenberger, Thomas in WO/2009/063333 Patent describes a composition that includes a carotenoid and either an anthocyanin, or a compound having at least one-SH group, or a combinations of both. He claims that when such composition is administered, the resulting bioavailability of the carotenoid is increased relative to a carotenoid composition that is without either the anthocyanin, or without the compound having at least one-SH group, or without the combination of both anthocyanin and a compound with at least one -SH group.

Madhavi, et al in US Patent 7,446,101 describes an improved commercial process for the production of carotenoid-cyclodextrin complexes and formulations of the complex for human ingestion. Complexation with cyclodextrins (alpha-cyclodextrin, beta-cyclodextrin, gamma-cyclodextrin, or HP-beta-cyclodextrin) significantly improves the uptake of carotenoids (lycopene, Lutein or Zeaxanthin) and their mixtures in vitro. In vivo, in a human study, the Lutein/gamma-cyclodextrin complex formulated in lecithin-oil or oil, showed a better absorption of Lutein as compared to the free Lutein-oil formulation.

Doney, John, Alfred, in WO/2008/080037 Patent describes carotenoid compositions of enhanced solubility and bioavailability that contain at least one solubility-enhancing polymer. In one embodiment the carotenoid is a provitamin A carotenoid such as β-carotene. In another embodiment the carotenoid is a non-provitamin A carotenoid such as lycopene or Lutein. The methods described to produce the bioenhanced products include dry blending and solvent spray drying. The method includes the steps providing a mixture comprising the carotenoid, a solubility enhancing-polymer and a solvent. The solvent is removed producing an amorphous form of the carotenoid. Products made by the invention's compositions and methods include pharmaceuticals, nutraceuticals, cosmetic, and personal care products for man and animal.

Kanner Joseph, et al, in US Patent 2008153148 discloses a method for increasing a fraction of free carotenoids in which at least some of the carotenoids are fatty acid esterified. The method is effected by contacting the source of esterified carotenoids with an effective amount of an esterase under conditions effective in deesterifying the fatty acid esterified carotenoids, thereby increasing the fraction of free carotenoids in the source.

In accordance with the present invention, micro micellar form of carotenoids or xanthophylls, which do not contain any crystal forms of carotenoids, obtained as micro micelles after melting Lutein diacetate or Lutein dipropionate in their natural original lipid vegetable matrix in the presence of lipids, phospholipids, fatty acids, emulsifiers and moisture, dosed at between 2 to 50 mg, to provide an absorption of the Lutein solubilizate which is at least 20% above than that of crystalline carotenoids, and provide a macular pigment deposit which exceeds at least 10% of Macular Pigment Optical Density, than the deposition obtained by ingesting crystalline Lutein. Thus preventing tissue degeneration, UV light damage to skin and the retina, and in general act as a more effective and efficient antioxidants than when administered in crystalline form.

### C.- SUMMARY OF THE INVENTION

It is therefore a main object of the present invention to prepare formulations and carotenoid containing compounds with improved absorption and bioavailability. Among such carotenoids are Lutein, 3'Epilutein, Zeaxanthin, Meso-Zeaxanthin, Astaxanthin, Cantaxanthin, Capsanthin, Capsorubin, and their derivatives that have been prepared as micro micelles according to US Pat.7,435,846, so their absorption and bioavailability are noticeably improved. The invention refers to any carotenoid that contains hydroxyl groups and/or pair of hydrogen atoms that are substituted by oxygen atoms.

It is also an object of the present invention to reduce the dose of a given carotenoid which has been formulated as micro micelles, in order to obtain the desired concentration level at a given tissue, as compared to carotenoids dosed in their crystalline form.

The carotenoids prepared as micro micelles may be used alone or in combination with other ingredients such as zinc, copper, selenium and manganese, as well as with vitamin A, vitamin C, and vitamin E, in order to produce a mixture with a synergistic effect, and to improved stability of the carotenoid in the gastrointestinal tract.

It is also an object of the present invention to prepare specific formulations containing micro micelle carotenoids in combination with fish or krill oils that contain omega three and omega 6 fatty acids.

Therefore it is a further object of the invention to obtain a desired concentration level of carotenoids in the bloodstream using a lower dose than that required when crystalline carotenoids are used.

It is also a further object of the invention to improve the absorption of the carotenoids through the intestine wall, and consequently reach a higher level of carotenoids in the bloodstream as compared to the level obtained when crystalline carotenoids are ingested.

The micro micellar carotenoids can be formulated in a liquid preparation, water or other edible liquid, or as edible oil dispersions, or as microcapsules, or dispersed in inert carriers.

### EXAMPLE

An evaluation (Richard A. Bone & John T. Landrum, FIU,2010) was carried to determine the bioavailability of a solubilizate form of Lutein which does not contain any crystal forms of carotenoids, obtained as micro micelles after melting Lutein diacetate or Lutein dipropionate in their natural original lipid vegetable matrix, in the presence of lipids, phospholipids, fatty acids, emulsifiers and moisture, herein referred as Micro-Mic™ Lutein in non-crystalline form as micro micelles vs. commercial crystalline Lutein. The evaluation was carried on for a period of 24 weeks with three groups of subjects that ingested daily one gelatin capsule containing the following:
- The first group ingested a capsule containing a dispersion of 20 mg of Micro-Mic™ in vegetable oil.
- The second group ingested a gelatin capsule containing a dispersion of 20 mg of commercial crystalline Lutein dispersed in vegetable oil.
- The third group ingested a gelatin capsule containing only vegetable oil (placebo).

The three gelatin capsules were all the same color (dark brown) .

Blood samples were collected weekly and the serum analyzed for Lutein by HPLC, and the concentration was reported as (ng/ul) of Lutein

### Micro-Mic group

| Subject # | Code | Baseline Lutein Conc. (ng/ul) A | Week 6 Lutein Conc. (ng/ul) B | Week 6/Basel ine | Week 12 Lutein Conc. (ng/ul) C | Week 12/ Baseline | Week 18 Lutein Conc. (ng/ul)D | Week 18/ Baseline | Week 24 Lutein Conc. (ng/ul) E | Week 24/ Baseli ne | Week 6-24/Base line |
|---|---|---|---|---|---|---|---|---|---|---|---|
| 3 | E1 | 0,133 | 1,132 | 8,543 | 1,747 | 13,185 | 1,918 | 14,476 | 1,110 | 8,372 | 11,144 |
| 28 | E10 | 0,073 | 0,549 | 7,504 | 0,310 | 4,246 | 0,339 | 4,640 | 0,252 | 3,452 | 4,961 |
| 32 | E11 | 0,109 | 0,297 | 2,722 | 0,254 | 2,329 | 0,355 | 3,257 | 0,175 | 1,608 | 2,479 |
| 30 | E12 | 0,061 | 0,690 | 11,364 | 0,320 | 5,276 | 0,144 | 2,365 | 0,577 | 9,502 | 7,127 |
| 6 | E2 | 0,084 | 0,225 | 2,666 | 0,293 | 3,472 | 0,236 | 2,801 | 0,178 | 2,108 | 2,762 |
| 4 | E3 | 0,104 | 1,000 | 9,612 | 1,241 | 11,931 | 0,983 | 9,450 | 0,994 | 9,555 | 10,137 |
| 9 | E4 | 0,128 | 0,402 | 3,129 | 0,309 | 2,405 | 0,405 | 3,151 | 0,223 | 1,736 | 2,605 |
| 12 | E5 | 0,082 | 0,286 | 3,489 | 0,255 | 3,116 | 0,217 | 2,643 | 0,214 | 2,616 | 2,966 |
| 17 | E6 | 0,077 | 0,635 | 8,282 | 0,607 | 7,908 | 0,439 | 5,728 | 0,516 | 6,727 | 7,161 |
| 19 | E7 | 0,099 | 0,572 | 5,796 | 0,504 | 5,103 | 0,604 | 6,116 | 0,388 | 3,928 | 5,236 |
| 21 | E8 | 0,116 | 0,836 | 7,182 | 0,612 | 5,265 | 0,474 | 4,076 | 0,430 | 3,695 | 5,054 |
| 25 | E9 | 0,136 | 0,387 | 2,848 | 0,867 | 6,385 | 0,554 | 4,079 | 0,708 | 5,214 | 4,631 |
| | | | | | | | | | | | |
| | **Average** | 0,100 | 0,584 | 6,095 | 0,610 | 5,885 | 0,556 | 5,232 | 0,480 | 4,876 | 5,522 |

### Lutein group

| Subject # | Code | Baseline Lutein Conc. (ng/ul) A | Week 6 Lutein Conc. (ng/ul) | Week 6/Baselin B e | Week 12 Lutein Conc. (ng/ul) C | Week 12/ Baseli ne | Week 18 Lutein Conc. (ng/ul)D | Week 18/ Baseli ne | Week 24 Lutein Conc. (ng/ul)E | Week 24/ Baseli ne | Week 6-24/Bas eline |
|---|---|---|---|---|---|---|---|---|---|---|---|
| 2 | M1 | 0,151 | 0,439 | 2,914 | 0,677 | 4,491 | 0,361 | 2,395 | 0,309 | 2,051 | 2,963 |
| 31 | M10 | 0,108 | 0,867 | 8,047 | 0,370 | 3,439 | 0,341 | 3,171 | 0,247 | 2,296 | 4,238 |
| 7 | M2 | 0,119 | 1,028 | 8,611 | 0,655 | 5,491 | 0,867 | 7,268 | 0,708 | 5,931 | 6,825 |
| 8 | M3 | 0,082 | 0,215 | 2,624 | 0,317 | 3,867 | 0,187 | 2,283 | 0,407 | 4,958 | 3,433 |
| 11 | M4 | 0,164 | 1,035 | 6,314 | 0,935 | 5,704 | 0,711 | 4,335 | 0,748 | 4,563 | 5,229 |
| 16 | M5 | 0,268 | 1,054 | 3,934 | 1,179 | 4,398 | 0,967 | 3,608 | 0,729 | 2,721 | 3,665 |
| 18 | M6 | 0,108 | 0,457 | 4,235 | 0,418 | 3,871 | 0,246 | 2,273 | 0,143 | 1,327 | 2,927 |
| 20 | M7 | 0,112 | 0,372 | 3,335 | 0,278 | 2,494 | 0,256 | 2,296 | 0,213 | 1,910 | 2,509 |
| 15 | M8 | 0,063 | 0,265 | 4,201 | 0,373 | 5,926 | 0,272 | 4,321 | 0,541 | 8,589 | 5,759 |
| 26 | M9 | 0,108 | 0,584 | 5,425 | 0,564 | 5,242 | 1,238 | 11,494 | 0,535 | 4,965 | 6,782 |
| | | | | | | | | | | | |
| | **Avera ge** | 0,128 | 0,632 | 4,964 | 0,577 | 4,492 | 0,545 | 4,345 | 0,458 | 3,931 | 4,433 |

### Placebo group

| Subjec t # | Code | Baseline Lutein Conc. (ng/ul) A | Week 6 Lutein Conc. (ng/ul) B | Week 6/Baseli ne | Week 12 Lutein Conc. (ng/ul) C | Week 12/ Baseline | Week 18 Lutein Conc. (ng/ul) D | Week 18/ Baseli ne | Week 24 Lutein Conc. (ng/ul)E | Week 24/ Baseline | Week 6-24/Bas eline |
|---|---|---|---|---|---|---|---|---|---|---|---|
| 5 | Q1 | 0,209 | 0,221 | 1,055 | 0,295 | 1,408 | 0,170 | 0,812 | 0,138 | 0,659 | 0,984 |
| 29 | Q10 | 0,104 | 0,100 | 0,962 | 0,060 | 0,576 | 0,027 | 0,261 | 0,065 | 0,624 | 0,606 |
| 10 | Q2 | 0,178 | 0,218 | 1,222 | 0,300 | 1,681 | 0,300 | 1,682 | 0,178 | 0,996 | 1,395 |
| 1 | Q3 | 0,177 | 0,342 | 1,940 | 0,133 | 0,755 | 0,127 | 0,722 | 0,107 | 0,608 | 1,006 |
| 13 | Q4 | 0,175 | 0,207 | 1,183 | 0,253 | 1,449 | 0,234 | 1,337 | 0,108 | 0,616 | 1,146 |
| 14 | Q5 | 0,236 | 0,253 | 1,072 | 0,240 | 1,018 | 0,169 | 0,716 | 0,161 | 0,682 | 0,872 |
| 22 | Q6 | 0,128 | 0,204 | 1,589 | 0,123 | 0,959 | 0,117 | 0,914 | 0,091 | 0,711 | 1,043 |
| 23 | Q7 | 0,087 | 0,145 | 1,672 | 0,099 | 1,135 | 0,137 | 1,576 | 0,072 | 0,831 | 1,303 |
| 24 | Q8 | 0,216 | 0,304 | 1,409 | 0,296 | 1,371 | 0,266 | 1,232 | 0,206 | 0,952 | 1,241 |
| 27 | Q9 | 0,163 | 0,141 | 0,863 | 0,151 | 0,923 | 0,098 | 0,598 | 0,093 | 0,571 | 0,739 |
| | | | | | | | | | | | |
| | **Avera ge** | 0,167 | 0,213 | 1,297 | 0,195 | 1,128 | 0,164 | 0,985 | 0,122 | 0,725 | 1,034 |

The results obtained are shown for Baseline date, week 6^{tn}, week 12^{th}, week 18^{th}, and week 24^{th}. The ratio between the concentrations at each week was divided by the Baseline concentration. The average of those ratios is reported at the base of each column.

### Summary

| | Baseline Lutein Conc. (ng/ul) A | Week 6 Lutein Conc. (ng/ul) B | Week 6/Basel ine | Week 12 Lutein Conc. (ng/ul) C | Week 12/Baseli ne | Week 18 Lutein Conc. (ng/ul)D | Week 18/Ba seline | Week 24 Lutein Conc. (ng/ul)E | Week 24/Bas eline | Week 6-24/Bas eline |
|---|---|---|---|---|---|---|---|---|---|---|
| **Micro-Mic group** | 0,100 | 0,584 | **6,095** | 0,610 | **5,885** | 0,556 | **5,232** | 0,480 | **4,876** | **5,522** |
| **Lutein group** | 0,128 | 0,632 | **4,964** | 0,577 | **4,492** | 0,545 | **4,345** | 0,458 | **3,931** | **4,433** |
| **Placebo group** | 0,167 | 0,213 | **1,297** | 0,195 | **1,128** | 0,164 | **0,985** | 0,122 | **0,725** | **1,034** |
| **Percentage difference between Micro-Mic** | | | | | | | | | | |
| **&** | **Lutein** | | 22,8% | | 31,0% | | 20,4% | | 24,0% | 24,6% |

The more relevant information is the summary, where the Micro-Mic™ is consistently above crystalline Lutein by 25°/-30 %.

### Rate of increase in MPOD in absorbance units per week

| | **Micro-Mic group** | **Lutein group** | **Placebo group** |
|---|---|---|---|
| **Mean** | 1,920E-03 | 1,690E-03 | -7,516E-04 |
| **Median** | 2,350E-03 | 1,550E-03 | 1,920E-04 |
| **St.** Dev. | 1,432E-03 | 1,749E-03 | 3,027E-03 |
| **Min** | 3,536E-05 | -8,000E-04 | -8,700E-03 |
| **Max** | 5,100E-03 | 5,100E-03 | 1,900E-03 |

### Micro-Mic group mean exceeds Lutein group mean by ∼ 14%

The serum results are a better indicator of bioavailability than the macular pigment results. It is well established that some subjects are serum responders, but macular pigment non-responders. However in this evaluation the Micro-Mic™ group mean MPOD (Macular Pigment Optical Density) exceeds that of the crystalline Lutein subjects by approximately 14%.

## Claims

1. A solubilizate form of a xanthophyll which does not contain any crystal forms of carotenoids, obtained as micro micelles after melting a xanthophyll diacetate or a xanthophyll dipropionate in their natural original lipid vegetable matrix in the presence of lipids, phospholipids, fatty acids, emulsifiers and moisture, in a dosage between 2 to 50 mg, to provide an absorption of the xanthophyll solubilizate which is at least 20% above than that of crystalline carotenoids, and provides an improved bioavailability in the macular pigment deposit which exceeds at least 10% of the Macular Pigment Optical Density, than the bioavailability obtained by ingesting crystalline xanthophylls.

2. The solubilizate form of a xanthophyll of claim 1, wherein the preferred dosage is between 8 and 10 mg.

3. The solubilizate form of a xanthophyll of claim 1, wherein the most preferred dosage is of 20 mg.

4. The solubilizate form of a xanthophyll of claim 1, which is dispersed in an oil rich in Omega 3 and Omega 6 fatty acids.

5. The solubilizate form of a xanthophyll of claim 1, wherein the solubilizate is a solubilizate of Lutein in the form of micro micelles that is better absorbed and is more bioavailable than the crystalline form.

6. The solubilizate form of a xanthophyll of claim 1, wherein the solubilizate is a solubilizate of 3'Epi Lutein in the form of micro micelles that is better absorbed and more bioavailable than the crystalline form.

7. The solubilizate form of a xanthophyll of claim 1, wherein the solubilizate is a solubilizate of 3R,3'R Zexanthin in the form of micro micelles.

8. The solubilizate form of a xanthophyll of claim 1, wherein the solubilizate is a solubilizate of 3R,3'R Zeaxanthin short chain diester dispersed in an oil rich in Omega 3 and Omega 6 fatty acids.

9. The solubilizate form of a xanthophyll of claim 1, wherein the solubilizate is a solubilizate of 3R,3'S Zeaxanthin (Meso-Zeaxanthin), in the form of micro micelles.

10. The solubilizate form of a xanthophyll of claim 1, wherein the solubilizate is a solubilizate of 3R,3'S Zeaxanthin (Meso-Zeaxanthin) short chain diester, dispersed in an oil rich in Omega 3 and Omega 6 fatty acids.

11. The solubilizate form of a xanthophyll of claim 1, wherein the solubilizate is a solubilizate of Capsanthin in the form of micro micelles.

12. The solubilizate form of a xanthophyll of claim 1, wherein the solubilizate is a solubilizate of Capsorubin, in the form of micro micelles.

13. The solubilizate form of a xanthophyll of claim 1, wherein the solubilizate is a solubilizate of Astaxanthin, in the form of micro micelles.

14. The solubilizate form of a xanthophyll of claim 1, wherein the solubilizate is a solubilizate of Astaxanthin short chain diester, dispersed in an oil rich in Omega 3 and Omega 6 fatty acids.

15. The solubilizate form of a xanthophyll of claim 1, wherein the solubilizate is a solubilizate of Cryptoxanthin short chain monoester in the form of micro micelles.

16. The solubilizate form of a xanthophyll of claim 1, wherein the solubilizate is a solubilizates of Lutein, 3'Epi-lutein, 3R,3'R Zeaxanthin, 3R,3'S Zeaxanthin (Meso-Zeaxanthin), Capsanthin, Capsorubin, Astaxanthin and Cryptoxanthin, in the form of micro micelles formulated in a liquid preparation, water or other edible liquid; or as edible oil dispersions; or as microcapsules; or dispersed in inert carriers.

17. The solubilizate form of a xanthophyll of claim 1, wherein the solubilizate is a solubilizate prepared as micro micelles, alone or in combination with other ingredients such as zinc, copper, selenium and manganese, as well as with vitamin A, Vitamin C, and Vitamin E, in order to produce a mixture with a synergistic effect, and to avoid degradation of the carotenoid in the gastrointestinal tract.

18. A process to use a solubilizate form of Lutein, 3R,3'R Zeaxanthin, 3R,3'S Zeaxanthin (Meso-Zeaxanthin), and Astaxanthin, in a micro micellar preparation, alone or in combination to prevent or treat maculopathy disorders, AMD, cataracts, and the like, comprising incorporating into the human body about 2 to 50 mg of such carotenoids, to prevent tissue degeneration, UV light damage to skin and the retina, and in general to act as more effective and efficient antioxidants than when administered in crystalline form.

19. A process to use a solubilizate of a xanthophyll, 3R,3'R Zeaxanthin, 3R,3'S Zeaxanthin (Meso- Zeaxanthin), Cantaxanthin and Astaxanthin in a micro micellar preparation, alone or in combination, comprising incorporating about 2 to 50 mg of such carotenoids in the organisms of poultry, marine species and crustaceans.
